# EUROPEAN PATENT APPLICATION

(11) **EP 3 847 957 A1**
(43) Date of publication of application: **14.07.2021**
(21) Application number: 21155722.8
(22) Date of filing: 10.10.2016
(51) Int. Cl.: A61B 5/022, A61B 17/132

(54) **WRIST-WORN BLOOD PRESSURE MONITOR**

(30) Priority: 08.10.2015 KR 20150141639; 16.08.2016 KR 20160103537
(62) Divisional of application: 16853955.9
(71) Applicant: Charmcare Co., Ltd., Geumcheon-gu, Seoul 08591 (KR)
(72) Inventor: LEE, Dong Hwa, 16895 Gyeonggi-do (KR)
(74) Representative: IPAZ

(57) **Abstract**

The present invention provides a wrist-worn blood pressure monitor configured to be worn around a wrist, comprising a wrist cuff (100) including a wrist strap (110) so as to be worn around a wrist; and a sensor (130) provided at the wrist cuff (100) in order to measure blood pressure wherein the wrist strap (110) includes a strap frame (111) made of a hard material and formed to be bent in a predetermined shape so as to be mounted to a predetermined portion of the wrist and to be worn around the predetermined portion of the wrist; and a connection strap (112) that is flexible and is secured to and supported by the strap frame.

## Description

### Technical field

The present invention relates to a wrist blood pressure monitor that is wearable on a wrist in order to measure blood pressure, and more particularly to a wrist-worn blood pressure monitor that is capable of automatically tightening a wrist cuff in order to compress a wrist.

### Background art

In general, blood pressure refers to the pressure of blood against the wall of a blood vessel, and a heart alternates between contraction and relaxation about 60 to 80 times a minute. The pressure applied to the blood vessel when the heart contracts to push out blood is called 'systolic blood pressure' and is also called 'maximal blood pressure' since the blood pressure is the highest at that time. Further, the pressure maintained in the blood vessel when the heart is relaxed to receive blood is called 'diastolic blood pressure' and is also called 'minimal blood pressure' since the blood pressure is the lowest at that time.

Usually, a normal person has a systolic blood pressure of 120 mmHg and a diastolic blood pressure of 80 mmHg. In Korea, at least one in four adults has high blood pressure, and this proportion rapidly increases after the age of 40, and, of course, there are also patients who suffer from low blood pressure.

High blood pressure becomes a problem since it can lead to life-threatening complications such as eye diseases, renal diseases, artery diseases, brain diseases and heart diseases if the high blood pressure is left untreated. Therefore, periodic measurement and management of blood pressure is needed for a hypertensive patient who is at risk for complications or has complications.

With recent increased concern over adult diseases attributable to high blood pressure and emphasis on health, various kinds of blood pressure measuring devices have been developed.

As a method of measuring the blood pressure, there are a Korotkoff sounds method, an oscillometric method, a tonometric method, etc.

The Korotkoff sounds method is a typical pressure measuring method, in which a body part where arterial blood flows is compressed enough to stop the flow of the blood and then the pressure at a moment at which a pulse first sounds is taken as systolic pressure and the pressure at a moment at which the pulse sound disappears is taken as diastolic pressure after the blood pressure has lowered.

The oscillometric method and the tonometric method are methods applicable to a digital blood pressure measuring device.

Like the Korotkoff sounds method, the oscillometric method presses the body part where the arterial blood flows with sufficient force to stop the flow of the arterial blood, after which, pulse waves, generated while the blood pressure is lowered at a constant speed or while the blood pressure of the body part is raised at a constant speed, are sensed, thereby taking the systolic pressure and the diastolic pressure.

In this method, pressure that has a constant level as compared with the maximum amplitude of the pulse wave may be taken as the systolic pressure or the diastolic pressure, or pressure when the pulse wave amplitude is rapidly changed may be taken as the systolic pressure or the diastolic pressure.

Further, while the body part is compressed and then decompressed at a constant speed, the systolic pressure is measured earlier than the moment at which the pulse wave has the maximum amplitude and the diastolic pressure is measured later than the moment at which the pulse wave has the maximum amplitude. On the other hand, while the compression is increased at a constant speed, the systolic pressure is measured later than the moment at which the pulse wave has the maximum amplitude and the diastolic pressure is measured earlier than the moment at which the pulse wave has the maximum amplitude.

Next, the tonometric method refers to a method of successively measuring the blood pressure based on the amplitude and shape of a pulse wave generated when a body part is compressed without completely stopping the flow of the arterial blood.

As described above, a blood pressure monitor for measuring the blood pressure is the most basic medical device for taking the blood pressure as a basic health index, and is thus not only almost essentially provided in general clinics and hospitals but is also frequently used in homes, at sport centers, etc. to measure personal blood pressure.

However, most conventional blood pressure monitors have been produced as a forearm style in which the blood pressure monitor is wound around an upper arm (or a portion from a shoulder to an elbow) and measures the blood pressure whenever there is a need to measure the blood pressure, which makes it inconvenient to carry and prevents a user from easily measuring the blood pressure at any desired time.

For example, because a conventional blood pressure monitor based on the oscillometric method needs a blood pressure monitor main body wound around and compressing a wrist or a forearm, a pressure cuff, a tube plug, an air hose, etc., it has shortcomings in that it has a complicated and bulky structure, and further it is cumbersome to use the conventional blood pressure monitor since there are many preparations for measurement, for instance, the air hose has to be fitted to face down for correct measurement, the cuff has to be mounted 1-2 cm higher than the elbow, and so on.

In particular, because the change in blood pressure varies depending on the physical features of the examinee, a patient who has complications has to check his/her blood pressure periodically and/or immediately when he/she feels sick; however, a mercury blood pressure monitor and an electronic blood pressure monitor are inconvenient for the patients due to the following problems.

First, the mercury blood pressure monitor and the electronic blood pressure monitor employ the cuff that requires air injection; however, the cuff is so bulky that it is cumbersome for a user to wear the cuff all the time.

In addition, the mercury blood pressure monitor employing a pneumatic cuff is so bulky and heavy that it is impractical for an examinee (i.e. a patient), who has to frequently measure his/her blood pressure, to always carry, and further it is cumbersome to attach and detach the blood pressure monitor whenever there is a need to check the blood pressure.

Although there has been proposed a pneumatic electronic blood pressure monitor having the same accuracy as the foregoing conventional blood pressure monitor, it is heavy and bulky since an electric pump and an air injection cuff are employed, which prevent a patient from moving freely if he/she has to carry the blood pressure monitor and periodically check his/her blood pressure.

In addition, there have been proposed pressureless electronic blood pressure monitors that measure the blood pressure based on pulses at a wrist or a fingertip and various other parameters; however, such a pressureless electronic blood pressure monitor has low precision since it is difficult to correctly specify and universally offer the parameters, and is difficult to apply to patients who have to periodically check their blood pressure with high accuracy.

In addition, a blood pressure monitor mounted to a wrist like a wristwatch and capable of measuring the blood pressure has recently been released, and is called a wrist blood pressure monitor, wrist-worn blood pressure monitor or wristwatch style blood pressure monitor.

Even though the conventional wrist-worn blood pressure monitor is called the wristwatch style for no other reason than that it is mounted to the wrist to measure the blood pressure, it is equipped with a mechanical/electronic pumping device, i.e. an air pump, to compress the wrist, which causes increased complexity in structure, difficulty in manipulation and operation, frequent failure of operation, and increased manufacturing costs. In addition, the conventional wrist-worn blood pressure monitor has operational and mechanical problems in that air has to be injected into an air bag to compress the wrist so that the wrist radial artery can be sufficiently compressed whenever a user checks his/her blood pressure, the accuracy of the measured blood pressure is degraded because the air bag is not brought into sufficiently close contact with the surface of the skin of the wrist, and an exhaust valve has to be employed in order to gradually exhaust air.

### Disclosure of the invention

The present invention has been made to solve the above problems, and it is an object of the present invention to provide a wrist-worn blood pressure monitor that is capable of being automatically tightened in order to compress a wrist with improved tightness of contact with the wrist and of being automatically untightened.

It is another object of the present invention to provide a wrist-worn blood pressure monitor that is capable of being adjusted in size (height) of a wrist cuff corresponding to the size of a wrist of a user.

In accordance with an aspect of the present invention, a wrist-worn blood pressure monitor which can be wearable on a wrist includes a wrist cuff including a wrist strap so as to be worn around a wrist, a sensor provided at the wrist cuff in order to measure blood pressure, and a tightener provided at the wrist cuff in order to selectively tighten the wrist cuff so that the wrist cuff compresses the wrist.

The wrist cuff may further include an air bag provided at the wrist strap in order to compress the wrist. And the air bag is connected with a valve for injection of air into the air bag.

At least one of the valve and the sensor is provided at the air bag.

And, the wrist strap may include a strap frame made of a hard material and formed to be bent in a predetermined shape so as to be mounted to a predetermined portion of the wrist and to be worn around the predetermined portion of the wrist, and a connection strap that is flexible and is secured to and supported by the strap frame. The strap frame forms a framework of the wrist cuff.

The connection strap extends from one end of the strap frame and is connected to an opposite end of the strap frame. More particularly, one end of the connection strap is secured to the strap frame, and an opposite end of the connection strap is detachably connected to the opposite end of the strap frame.

The opposite end of the connection strap and the opposite end of the strap frame can be connected to each other by the tightener.

The strap frame may include a base frame for supporting the connection strap, and an auxiliary frame for height adjustment, the auxiliary frame being movably provided at the base frame in order to adjust a height of the wrist cuff to match a size of the wrist.

The strap frame has a curved shape so as to cover a portion corresponding to an ulnar artery. The air bag is provided at the connection strap.

The tightener may includes a cuff connector for connecting one end portion of the wrist cuff to an opposite end portion of the wrist cuff, and a driving unit connected to the cuff connector in order to apply pulling force to an opposite end portion of the wrist cuff via the cuff connector.

The driving unit may include a motor provided at the wrist cuff, a roll rotatably provided at the wrist cuff in order to wind the cuff connector therearound, and a gear train for transmitting rotating force of the motor to the roll.

The cuff connector can be detachably connected to the opposite end portion of the wrist cuff. The air bag may have an airtight structure such that air is prevented from being injected thereinto.

The tightener may include a cuff connector movably provided at one end portion of the wrist cuff in order to connect the one end portion of the wrist cuff to an opposite end portion of the wrist cuff so that the wrist cuff forms a closed loop, and a pulling device provided at the wrist cuff in order to selectively pull the cuff connector to tighten the wrist cuff. The cuff connector and the pulling device can be configured to be connected to each other or disconnected from each other.

The pulling device may include a pulling rod connected to the cuff connector, and an actuator for moving the cuff connector along the wrist cuff by driving the pulling rod. And the pulling rod includes a screw bar configured to be rotated by the actuator, to have screw threads formed in an outer circumferential surface thereof, and to be rotatably inserted into the cuff connector, and the cuff connector is configured to be moved along the pulling rod.

The cuff connector is selectively engaged with the pulling rod so as to be pulled by the pulling device.

The cuff connector may include a connector body provided at the one end portion of the wrist cuff in order to connect the one end portion of the wrist cuff to the opposite end portion of the wrist cuff, having a rod hole formed therethrough, into which the pulling rod is inserted, and configured to be moved along the pulling rod, and a rod connection/disconnection member movably provided at the connector body for connection and disconnection between the cuff connector and the pulling rod and configured to selectively mesh with the screw threads in the pulling rod.

The connector body may include an electromagnet for moving the rod connection/disconnection member so that the rod connection/disconnection member meshes with the screw threads in the pulling rod.

When supply of power to the electromagnet is interrupted, the rod connection/disconnection member is separated from the pulling rod. The rod connection/disconnection member can be separated from the pulling rod by restoring force of a spring.

In accordance with another aspect of the present invention, a wrist-worn blood pressure monitor includes a wrist cuff including a wrist strap so as to be worn around a wrist, a sensor provided at the wrist cuff in order to measure blood pressure, and a tightener provided at the wrist cuff in order to selectively tighten the wrist cuff so that the wrist cuff compresses the wrist, the tightener including a cuff connector provided at the wrist cuff in order to connect one end portion of the wrist cuff to an opposite end portion of the wrist cuff, and a driving unit connected to the cuff connector in order to apply pulling force to the opposite end portion of the wrist cuff via the cuff connector.

The cuff connector may be detachably connected to the opposite end portion of the wrist cuff. The wrist cuff may further include an air bag provided at the wrist strap in order to locally compress the wrist, and the air bag may be connected with a valve for injection of air thereinto.

In accordance with a further aspect of the present invention, a wrist-worn blood pressure monitor includes a wrist cuff including a wrist strap so as to be worn around a wrist, a sensor provided at the wrist cuff in order to measure blood pressure, and a tightener provided at the wrist cuff in order to selectively tighten the wrist cuff so that the wrist cuff compresses the wrist, the wrist strap including a strap frame made of a hard material and formed to be bent in a predetermined shape so as to be mounted to a predetermined portion of the wrist and to be worn around the predetermined portion of the wrist, and a connection strap that is flexible and is secured to and supported by the strap frame. The wrist cuff may further include an air bag provided at the wrist strap in order to locally compress the wrist, and the air bag may be connected with a valve for injection of air thereinto.

A wrist-worn blood pressure monitor according to an embodiment of the present invention has the following advantages.

First, according to an embodiment of the present invention, a user is able to conveniently measure his/her blood pressure whenever there is a need to measure the blood pressure, and the accuracy of the measured blood pressure is increased since the wrist-worn blood pressure monitor is securely tightened around the wrist.

In addition, according to an embodiment of the present invention, the wrist-worn blood pressure monitor is capable of being adjusted in size (height) corresponding to the size of a wrist of a user while maintaining the shape thereof and of ensuring blood flow in an ulnar artery while blood pressure measurement is being conducted.

Further, according to an embodiment of the present invention, since it is possible to supplement the air in the air bag depending on a change in the external environment, for example, temperature change, deterioration in the performance of the wrist-worn blood pressure monitor is prevented.

Further, it is important to measure and record blood pressure at the same time every day in order to determine a pattern of changing blood pressure, and according to an embodiment of the present embodiment, the wrist-worn blood pressure monitor is very useful in periodically and automatically measuring the blood pressure using an electronic automatic control process, and is very advantageous in regularly measuring the blood pressure without forgetting if an alarm is given.

Besides, according to the present invention, data of the measured blood pressure may be sent to an attending physician or a medical specialist through a wireless communication unit and then analyzed, so that it can be more effective to patients with high blood pressure, diabetes, hepatic impairment, hardening of arteries, peripheral nerve disorder of blood circulation, etc.

Further, according to an embodiment of the present invention, since the wrist-worn blood pressure monitor may be configured so as to have a sealed air bag without the need for a pumping device for filling the air bag with air, an exhaust device for exhausting air, for example, an exhaust valve, is not needed either.

Furthermore, according to an embodiment of the present invention, the wrist-worn blood pressure monitor is capable of being naturally untightened without using additional power and of being naturally loosened or untightened when a battery dies during blood pressure measurement, thereby providing improved safety in use and preventing blood flow disturbance or other accidents due to the wrist-worn blood pressure monitor.

### Description of Drawings

The features and advantages of the present invention will be more clearly understood from the following detailed description taken in conjunction with the accompanying drawings, in which:
**[****Fig. 1****]** is a side view schematically illustrating an embodiment of a wrist-worn blood pressure monitor according to the present invention;
**[****Fig. 2****]** is a sectional view illustrating a wrist cuff of the wrist-worn blood pressure monitor depicted in FIG. 1;
**[****Fig. 3****]** is a side view illustrating the state in which the wrist-worn blood pressure monitor depicted in FIG. 1 forms a closed loop;
**[****Fig. 4****]** is a view schematically illustrating an embodiment of a cuff tightener that is applicable to the wrist-worn blood pressure monitor depicted in FIG. 1;
**[****Fig. 5****]** is a block diagram illustrating the overall construction of the wrist-worn blood pressure monitor according to the present invention;
**[****Fig. 6****]** is a sectional view schematically illustrating another embodiment of the wrist-worn blood pressure monitor according to the present invention;
**[****Fig. 7****]** is a top planar view of the wrist-worn blood pressure monitor depicted in FIG. 6;
**[****Fig. 8****]** is a sectional view illustrating the state in which the wrist-worn blood pressure monitor depicted in FIG. 6 is worn around a wrist and forms a closed loop;
**[****Fig. 9****]** is a sectional view illustrating the state in which the wrist-worn blood pressure monitor depicted in FIG. 6 is tightened in order to compress a wrist;
**[****Fig. 10****]** is a perspective view illustrating a portion of a wrist cuff employed in the wrist-worn blood pressure monitor depicted in FIG. 9;
**[****Fig. 11****]** is a side view illustrating an embodiment of a tightening device for automatically tightening the wrist cuff (i.e. a cuff tightener) according to the present invention;
**[****Fig. 12****]** is a sectional view illustrating an embodiment of a cuff connector for the wrist-worn blood pressure monitor according to the present invention;
**[****Fig. 13****]** is a perspective view of the cuff connector depicted in FIG. 12;
**[****Fig. 14****]** is a perspective view for explaining connection and disconnection between the cuff connector depicted in FIG. 11 and a pulling rod; and
**[****Fig. 15****]** is a sectional view illustrating the operation of the cuff connector depicted in FIG. 12.

### [Best Mode]

Hereinafter, exemplary embodiments of the present invention will be described in detail with reference to the accompanying drawings. The same elements are designated by the same reference numerals throughout the specification.

The modes for implementing the present invention, i.e. the embodiments of the present invention, relate to a device for measuring blood pressure by tightening a wrist cuff for compressing a wrist, i.e. a portable blood pressure measuring device in which the wrist cuff is contracted to compress the wrist.

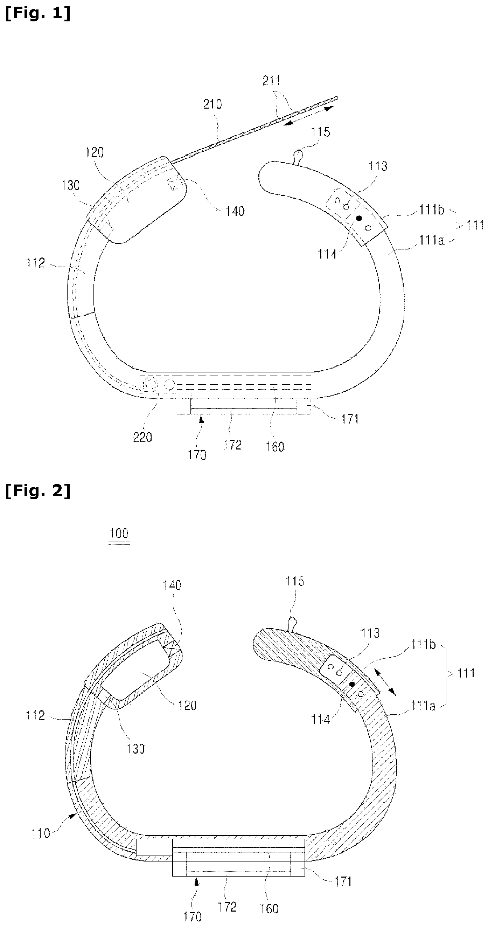

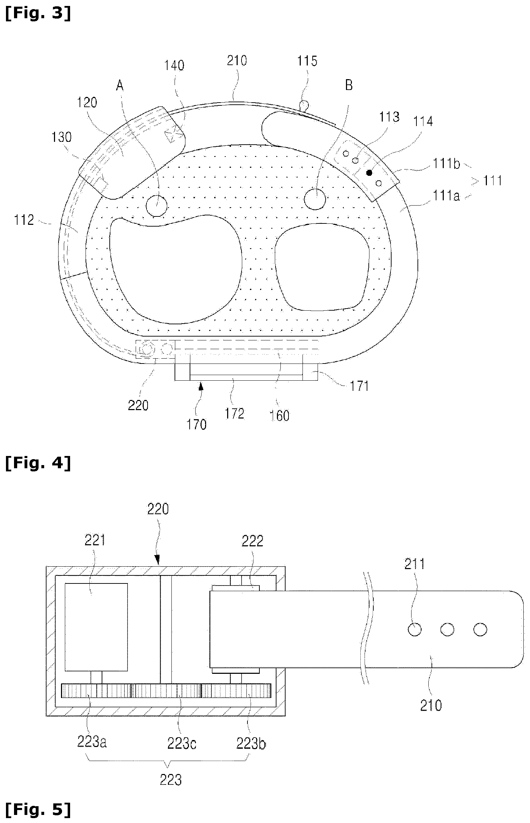

First, an embodiment of a wrist-worn blood pressure monitor according to the present invention will be explained with reference to FIGS. 1 to 4. FIG. 1 is a side view schematically illustrating an embodiment of a wrist-worn blood pressure monitor according to the present invention, FIG. 2 is a sectional view illustrating a wrist cuff of the wrist-worn blood pressure monitor depicted in FIG. 1, FIG. 3 is a side view illustrating the state in which the wrist-worn blood pressure monitor depicted in FIG. 1 forms a closed loop, and FIG. 4 is a view schematically illustrating an embodiment of a cuff tightener that is applicable to the wrist-worn blood pressure monitor depicted in FIG. 1.

Referring to FIGS. 1 to 4, a wrist-worn blood pressure monitor (wrist blood pressure monitor) according to this embodiment (a first embodiment) is a blood pressure monitor that can be worn around a wrist and that includes a wrist cuff 100 configured to be worn around the wrist and a cuff tightener 200 for tightening the wrist cuff 100.

The wrist cuff 100 is configured to be worn around the wrist like a wristwatch and includes a wrist strap 110 for fastening the wrist cuff around the wrist, and a sensor 130 for measuring the blood pressure is provided at the wrist cuff 100.

The wrist cuff 100 may further include an air bag 120 for compressing a predetermined portion of the wrist, for example, a wrist radial artery, and, in this embodiment, the air bag 120 is provided at the wrist strap 110 and the sensor 130 is connected with the air bag 120.

In other words, the wrist-worn blood pressure monitor according to this embodiment is a blood-pressure-measuring cuff, which is worn around the wrist, so that a user can carry it like a wristwatch, and which enables a patient in bed, who is not able to move freely, to wear it around his/her wrist and to check his/her blood pressure periodically and/or immediately when he/she wants to do it, and which may be connected with other devices, for example, a blood pressure maintenance monitor, over a wired or wireless medium.

The wrist strap 110 has a band or a belt configuration, which is worn around the wrist, the air bag 120 is provided at the wrist strap 110 in order to compress the wrist, and the sensor 130 in this embodiment is provided at the air bag 120 in order to sense the change in pressure of the air bag 120; however, the embodiment of the present invention is not limited to these configurations.

The air bag 120 is configured to compress the wrist in order to measure pulse pressure from the wrist, and may have a structure such that air injection into the air bag is not needed, i.e. a sealed structure; however, in this embodiment, the air bag 120 may have a structure such that air can be injected into the air bag, for example, a structure that is connected with a valve 140.

The valve 140 may be embodied as a check valve and may be connected with the air bag 120 through a conduit, for example, a tube, etc.; however, in this embodiment, the valve 140 is provided at the air bag 120. Therefore, in this embodiment, the sensor 130 and the valve 140 are mounted to the air bag 120, and when the internal pressure in the air bag 120 is lower than the external pressure, i.e. atmospheric pressure, supplemental air can be provided to the air bag 120 via the valve 140.

The air bag 120 compresses the surface of the skin of the wrist, for example, a portion corresponding to a radial artery A, and in this embodiment, the sensor 130 may be embodied as a device for sensing the pressure of the air bag 120, for example, a pressure sensor. Various kinds of sensors capable of measuring pneumatic pressure may be employed as the sensor 130, and in this embodiment, the sensor 130 is connected with a control unit, i.e. a circuit board 160 for calculating the blood pressure, through a cable (not shown) mounted to the wrist strap 110.

The wrist strap 110 may be made of a flexible material such as leather, fabric, synthetic resin, etc., and the air bag 120 may be made of an elastic material such as silicon, urethane, rubber, etc.

In this embodiment, the wrist cuff 100 forms a closed loop such that one end portion and the opposite end portion of the wrist cuff 100 are detachably connected to each other, and the wrist strap 110 may be divided into a left strap and a right strap by a display unit 170, for displaying the blood pressure, interposed therebetween. Of course, the wrist cuff 100 may have an integral structure, i.e. a circulation structure, in which the wrist cuff 100 is continuously extended without a detachable connector, and may be tightened by the cuff tightener 200.

In this embodiment, the wrist strap 110 includes a strap frame 111 and a connection strap 112. The strap frame 111 is formed of a hard material and is formed to be bent in a predetermined shape so as to be mounted to a predetermined portion of the wrist, in particular, so as to be worn around the ulna of the wrist, and functions as a frame of the wrist cuff 100.

That is, the strap frame 111 may be made of a material having hardness greater than a predetermined level, for example, metal or plastic, so as to secure stability in shape. The connection strap 112 is a flexible strap, which is secured to and supported by the strap frame 111 and functions to bridge the gap between one end and the opposite end of the strap frame 111.

Described in more detail, the connection strap 112 extends from one end portion of the strap frame 111 and is connected to the opposite end portion of the strap frame 111, and may be made of a material having flexibility and being capable of being easily bent, for example, rubber, silicon, fabric, leather, etc.

In this embodiment, one end of the connection strap 112 is secured to the strap frame 111, and the opposite end of the connection strap 112 is detachably connected to the opposite end of the strap frame 111. Described in more detail, the opposite end of the connection strap 112 and the opposite end of the strap frame 111 are indirectly connected to each other by the cuff tightener 200.

Of course, at least a portion of the strap frame 111 may be disposed inside the connection strap 112 so as to be covered with the connection strap 112.

The air bag 120 is provided at the connection strap 112, and the connection strap is detachably connected to the opposite end of the strap frame 111 by the cuff tightener 200. The strap frame 111 has a curved shape so as to cover a portion corresponding to the ulnar artery B.

Therefore, according to this embodiment, when the wrist cuff 100 is worn around the wrist so that the strap frame 111 covers a portion corresponding to the ulnar artery B, the wrist cuff 100 is tightened by the cuff tightener 200 and the blood pressure measurement is performed in a tonometric method while allowing the flow of the blood in the ulnar artery.

The strap frame 111 may include a base frame 111a and an auxiliary frame 111b. The base frame 111a is secured to the connection frame and supports the connection strap 112.

The auxiliary frame 111b is a frame for height adjustment, and is movably connected to the base frame 111a so as to adjust the height of the wrist cuff 100 to match the size of the wrist. Accordingly, the strap frame 111 forms a frame having an extendable and contractible structure.

Although the base frame 111a and the auxiliary frame 111b may be made of the same material or different materials from each other, they must be manufactured to have predetermined strength sufficient to form the frame of the wrist cuff 100.

Referring to FIGS. 1 to 3, the auxiliary frame 111b has a curved shape, for example, is curved at an obtuse angle or is curved corresponding to the shape of the surface of the skin at the ulna of the wrist, and is movably assembled with the base frame 111a.

The auxiliary frame 111b may be slidably fitted into the base frame 111a; however, in this embodiment, the base frame 111a is fitted into the auxiliary frame 111b.

The auxiliary frame 111b has a plurality of assembly holes 113 for height adjustment formed to be spaced apart from each other in the longitudinal direction of the auxiliary frame 111b, and the base frame 111a is provided with at least one assembly pin 114, which is elastically biased in the outward direction of the base frame 111a by an elastic member (not shown), such as a spring, etc., mounted in the base frame 111a.

Therefore, the assembly pin 114 is coupled to any one of the assembly holes 113, and the height of the wrist cuff 100, more specifically, the height of the strap frame 111, is adjusted by varying the coupling position of the assembly pin 114.

The wrist cuff 100 may further include a display unit 170 for displaying (outputting) the blood pressure of the user (or the examinee), i.e. the wearer, and the aforementioned circuit board 160 is mounted in the display unit 170.

The display unit 170 has a function of displaying the blood pressure, and may further have the function of a wristwatch. For example, the display unit 170 may be operated to alternate between the blood pressure output mode and the wristwatch mode or to simultaneously display the blood pressure and the time.

In this embodiment, the display unit 170 includes a case 171, which accommodates electronic components, and a display window 172 provided at the case 170, and the case 171 is mounted to the wrist strap 110. The blood pressure may be displayed through the display window 172, and the time may be further displayed through the display window 172.

The circuit board 160 is electrically connected with the sensor 130, thereby calculating the blood pressure and outputting the value of the blood pressure to the display window 172.

Although not illustrated in the drawings, a battery for supplying electric power to the wrist-worn blood pressure monitor may be mounted in the case 171. Alternatively, the wrist-worn blood pressure monitor according to this embodiment may receive electric power from the outside through a power cable, or may be operated using sunlight.

Further, the display unit may have an alarm function, for example, a function of generating a notification indicating the blood pressure measurement time and/or a notification indicating that the blood pressure measurement has been completed normally. Accordingly, a user is able to measure and record his/her blood pressure accurately when there is a need to check the blood pressure. In this embodiment, the display unit 170 is mounted to the strap frame 111.

As described above, the wrist-worn blood pressure monitor may periodically or occasionally perform the blood pressure measurement depending on the user manipulation; however, the blood pressure measurement may be automatically performed at regular or irregular time intervals under the control of the circuit board 160, i.e. a control module for controlling the operation of the wrist-worn blood pressure monitor.

One end portion and the opposite end portion of the wrist cuff 100 may be connected to each other using a fastening means, such as Velcro, a hook, a button, a buckle, etc., and in this embodiment, the wrist cuff 100 has a loop configuration due to the cuff tightener 200. Of course, the wrist cuff 100 may have a closed-loop structure in which the wrist cuff 100 is continuously extended, i.e. an integral band structure.

The cuff tightener 200 is configured to tighten the wrist cuff 100 and is provided at the wrist cuff 100. Described in more detail, the cuff tightener 200 tightens the wrist cuff 100 so that the air bag 120 compresses a predetermined portion of the wrist, i.e. a portion corresponding to the radial artery.

In other words, the cuff tightener 200 is configured to tighten the wrist cuff 100 of the closed-loop state, specifically, the wrist strap 110. The cuff tightener 200 is a device for tightening the wrist-worn blood pressure monitor by reducing the peripheral length of the wrist cuff 100, i.e. a device for automatically tightening the wrist cuff 100 so that the wrist of the examinee, specifically, a portion corresponding to the radial artery A, may be compressed at a predetermined pressure or more for the blood pressure measurement in the state in which the wrist-worn blood pressure monitor is worn around the wrist of the examinee.

The cuff tightener 200 may employ various kinds of electrically driven devices, such as a linear motor, a rack-and-pinion device, a cylinder device, a motor-driven reel, etc.; however, in this embodiment, the cuff tightener 200 includes a cuff connector 210 and a driving unit 220.

The cuff connector 210 functions to connect one end portion of the wrist cuff 100 to the opposite end portion of the wrist cuff 100, and accordingly the wrist cuff 100 forms a closed loop. In this embodiment, the cuff connector 210 detachably connects one end portion of the wrist cuff 100 to the opposite end portion of the wrist cuff 100.

The driving unit 220 is provided at the wrist cuff 100 in order to apply pulling force to the opposite end portion of the wrist cuff 100. In more detail, the driving unit 220 applies pulling force to the opposite end portion of the wrist cuff 100 via the cuff connector 210, and the cuff connector 210 is connected to the driving unit 220 in order to pull the opposite end portion of the wrist cuff 100 toward one end portion of the wrist cuff 100, thereby tightening the wrist cuff 100.

In this embodiment, the cuff connector 210 has a flexible configuration, such as a string, a strap, a band, etc., and is extended from one end portion of the wrist cuff 100. To this end, one end portion of the cuff connector 210 is secured to the wrist cuff 100, and the opposite end portion of the cuff connector 210 is detachably connected to the opposite end portion of the wrist cuff 100.

Described in more detail, the cuff connector 210 is extended toward the outside of the connection strap 112 and is detachably connected to the opposite end portion of the strap frame 111, specifically to the auxiliary frame 111b.

The cuff connector 210 has at least one strap connection hole 211 formed therein, and the wrist cuff, specifically the auxiliary frame 111b, is provided with an engagement protrusion 115, such as a pin, a hook, etc., which is fitted into the strap connection hole 211. In order to secure the stable connection of the cuff connector 210, the engagement protrusion 155 has a head formed at the top thereof, which is enlarged larger than the strap connection hole 211.

The cuff connector 210, i.e. the strap connection unit, is movably provided at one end portion of the wrist strap, more specifically at the right strap 112, and the wrist strap 110 has a movement passage formed therein, through which the cuff connector 210 moves.

The driving unit 220 is configured to tension the wrist cuff 100 to a certain degree or more for the blood pressure measurement by applying pulling force to the cuff connector 210.

The driving unit 220 includes a motor 221, a winding roll 222, and a power transmission 223. The motor 221 is secured to the wrist cuff 100, and in this embodiment, is mounted to the strap frame 111, specifically to the base frame 111a. More particularly, the motor 221 is mounted in the base frame 111a, and may be connected to the battery mounted to the display unit so as to be operated by the battery.

The winding roll 222 is rotatably provided at the wrist cuff 100 so that the cuff connector 210 may be wound around the winding roll 222, and is rotated by the power transmitted thereto from the motor 221. As the result of the cuff connector 210 being wound around the winding roll 222, the wrist cuff 100 is tightened.

The power transmission 223 is configured to transmit power of the motor 221, i.e. rotating force thereof, to the winding roll 222, and, in this embodiment, includes a gear train. The power transmission 223, i.e. the gear train, includes a driving gear 223a, which is coupled to a shaft of the motor 221, and a driven gear 223b, which is coupled to a shaft of the winding roll 222 and is configured to be driven and rotated by the driving gear, and may further include a counter gear 223c, which is interposed between the driving gear 223a and the driven gear 223b in order to connect them. The power transmission 223 is not limited to the above-described gear train, and may alternatively employ a chain belt system or a timing belt system.

The driving unit 220 is provided at the strap frame 111, specifically at the base frame 111a, and the cuff connector 210 is mounted along the connection strap 112. In this embodiment, the cuff connector 210 is mounted in the connection strap 112 so as to be inserted into and drawn out of the connection strap 112, and the air bag 120 is provided at the connection strap 112.

Although not illustrated in the drawings, the wrist cuff 100 is provided with a switch for operating the cuff tightener. For example, the switch for operating the cuff tightener 200 is provided at the display unit 170, and when the switch is pressed, the cuff tightener is operated and the blood pressure measurement is started.

The motor 221 may be embodied as a motor that is capable of rotating in forward and reverse directions, and the operation of the motor 221 may be controlled by the control module of the foregoing circuit board 160. Therefore, when the blood pressure measurement is completed, the cuff connector 210 is released by the motor 221 and the wrist cuff 100 is loosened. Since the technology associated with the operation and control of the motor 221 is well known, further explanation thereof is omitted.

The cuff connector 210 may be wound around the winding roll 222 using the power of the motor 221, and the cuff connector 210 may be unwound from the winding roll 222 in a no-load state, in which the supply of electric power to the motor 221 is interrupted.

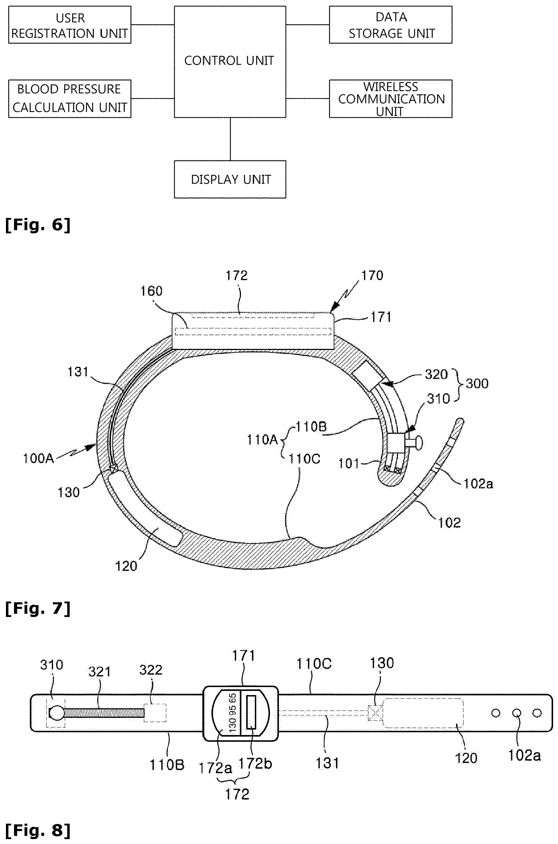

Referring to FIG. 5, in addition to the foregoing display unit, the wrist-worn blood pressure monitor according to this embodiment may further include a wireless communication unit such as a Bluetooth unit or the like for transmitting data of the measured blood pressure (the blood pressure of the user) to a predetermined terminal, for example, a smart phone, other kinds of terminals, or a management module.

The wireless communication unit may be configured to transmit a distress signal in a certain situation, for example, an emergency situation, to an external communication device as well as the data of the measured blood pressure. Accordingly, the data of the measured blood pressure of the examinee (the user) may be sent in real time to a manager, such as an attending physician or a medical specialist, through a smart phone, and may then be utilized for stable heath management of the examinee.

That is, more care is needed for persons with high blood pressure, diabetes, hepatic impairment, hardening of arteries, peripheral nerve disorder of blood circulation, etc., and thus the blood pressure monitor in this embodiment is particularly efficient in managing the blood pressure of such patients since it has a function of transmitting data.

The wrist-worn blood pressure monitor may include a blood pressure calculation unit for calculating the blood pressure of the examinee based on the signal from the pressure sensor, a data storage unit for storing data of the measured blood pressure, a user registration unit for managing a user's measurement history and a user's identification (ID), and a control unit for collecting and analyzing data and controlling a designated distress signal to be transmitted when a user is in an emergency situation.

The present invention is not limited to the above embodiment and may be modified and varied without departing from the scope of the invention, and the above embodiment is to be construed as illustrative and not restrictive.

### [Mode for Invention]

Hereinafter, another embodiment (a second embodiment) of the wrist-worn blood pressure monitor according to the present invention will be described, and in the following description of the second embodiment of the present invention, elements the same as those in the first embodiment are denoted by the same reference numerals.

Referring to FIGS. 6 to 9, the wrist-worn blood pressure monitor according this embodiment is a blood pressure monitor, which is configured to be worn around the wrist and to compress the wrist by being tightened by contraction, like the first embodiment, and which includes a wrist cuff 100A, which may be worn around the wrist in the form of loop, a sensor 130 for sensing the blood pressure, and a cuff tightener 300.

Similar to the previous embodiment (the first embodiment), the wrist-worn blood pressure monitor according to this embodiment is a blood-pressure-measuring cuff, which is worn around the wrist in order to measure the blood pressure and may have a portable configuration that a user can carry it like a wristwatch, and which enables a patient in bed, who is not able to move freely, to wear it around his/her wrist and to check his/her blood pressure periodically and/or immediately when he/she wants to do it, and which may be connected with other devices over a wired or wireless medium.

The wrist cuff 100A includes a wrist strap 110A so as to be worn around the wrist, and in this embodiment, the wrist strap 110A has a band or belt configuration that is worn around the wrist. The wrist cuff further includes an air bag 120, which is provided at the wrist strap 110A in order to compress the wrist.

The air bag 120 is configured to compress the wrist in order to measure pulse pressure from the wrist, and may have a structure in which air can be injected into and exhausted from the air bag, for example, an air chamber configuration having an air injection hole and an air exhaust hole; however, in this embodiment, the air bag 120 has a structure in which air injection into the air bag 120 is not needed, i.e. a sealed structure in which the air bag is always filled with air.

In this embodiment, the air bag 120 locally compresses the surface of the skin of the wrist, for example, a portion corresponding to a radial artery. The sensor 130 may be embodied as a device for sensing the pressure of the air bag 120, for example, a pressure sensor. Various kinds of sensors capable of measuring pneumatic pressure may be employed as the sensor 130, and the sensor 130 is provided at the air bag 120 and is connected with a control unit, i.e. a circuit board 160 serving as a blood-pressure-calculating unit, through an electric wire, i.e. a cable 131 mounted to the wrist strap 110.

The wrist strap 110A may be made of a flexible material such as leather, fabric, silicon, etc., and the air bag 120 may be made of an elastic material such as silicon, urethane, rubber, etc.

In this embodiment, the wrist strap 110A forms a closed loop such that one end portion and the opposite end portion of the wrist strap 110A are detachably connected to each other, and the wrist strap 110A is provided with a display unit 170. In this embodiment, the wrist strap 110A may be divided into a left strap 110B and a right strap 110C by the display unit 170 interposed therebetween.

One end portion and the opposite end portion of the wrist cuff 100, specifically one end portion and the opposite end portion of the wrist strap 110A, may be connected to each other by a strap connection unit, such as Velcro, a hook, a button, a buckle, etc., and in this embodiment, the cuff tightener 300 performs the connection of the wrist strap. Of course, the wrist cuff may have a closed-loop structure such that an additional detachable connection device is not needed. For example, the wrist strap may have an integral band structure in which the wrist strap is continuously extended without a detachable connection device.

The display unit 170 has a function of displaying the blood pressure, and may further have the function of a wristwatch. For example, the display unit 170 may be operated to alternate between the blood pressure output mode and the wristwatch mode or to simultaneously display the blood pressure and the time.

In this embodiment, the display unit 170 includes a case 171, which accommodates electronic components, and a display window 172, which includes a blood pressure display portion 172a for displaying the blood pressure and a time display portion 172b for outputting the time.

A circuit board 160, which is electrically connected with the sensor 130 in order to calculate the blood pressure and output the value of the blood pressure to the blood pressure display window, may be mounted in the case 171. In this embodiment, the circuit board 160, as described above, serves as a control unit, i.e. a blood pressure calculation unit.

Although not illustrated in the drawings, a battery for supplying electric power to the wrist-worn blood pressure monitor may be mounted in the case 171, and the circuit board 160 may control the operation of the cuff tightener 300 proposed in this embodiment. Alternatively, the wrist-worn blood pressure monitor according to this embodiment may receive electric power from the outside through a power cable, or may be operated using sunlight.

Further, the display unit may have an alarm function, for example, a function of generating a notification indicating the blood pressure measurement time and/or a notification indicating that the blood pressure measurement has been completed normally. Accordingly, a user is able to measure and record his/her blood pressure accurately when there is a need to check the blood pressure.

As described above, the wrist-worn blood pressure monitor according to this embodiment may periodically or occasionally perform blood pressure measurement in response to user manipulation; however, the blood pressure measurement may be automatically performed at regular or irregular time intervals by the circuit board 160, i.e. the control unit.

The cuff tightener 300 is configured to tighten the wrist-worn blood pressure monitor of the closed-loop state, specifically, the wrist cuff 100A, and more particularly, is a device for tightening or contracting the wrist-worn blood pressure monitor by reducing the peripheral length of the wrist cuff 100A.

In other words, the cuff tightener 300, like the above-described first embodiment, is a device for selectively tightening the wrist cuff 100A, and more particularly, is a device for automatically tightening the wrist cuff 100A so that the wrist of the examinee, specifically, a portion corresponding to the radial artery, may be compressed at a predetermined pressure or more by the air bag 120 for blood pressure measurement in the state in which the wrist-worn blood pressure monitor is worn around the wrist of the examinee.

The cuff tightener 300 may employ various kinds of electrically driven devices, such as a linear motor, a rack-and-pinion device, a cylinder device, a motor-driven reel, etc.; however, in this embodiment, the cuff tightener 300 includes a cuff connector 310 and a pulling device 320.

The cuff connector 310 functions to detachably connect one end portion 101 of the wrist cuff 100A to the opposite end portion 102 of the wrist cuff, and the wrist cuff 100A, specifically, the wrist strap 110A, forms a closed loop due to the cuff connector 310.

In this embodiment, the cuff connector 310 has a pin configuration that is fitted into at least one strap connection hole 102a, which is formed in the opposite end portion 102 of the wrist cuff. The cuff connector 310 includes an engagement protrusion 310a, which is formed at the upper portion of the cuff connector 310 so as to be fitted into the strap connection hole 102a, and the engagement protrusion 310a has a latching head formed at the top thereof, which is enlarged larger than the strap connection hole 102a so as to prevent separation of the strap.

The cuff connector 310, i.e. the strap connection unit, is movably provided at the wrist cuff 100A, more specifically at the left strap 110B, and the wrist cuff 100A has a movement passage formed therein, through which the cuff connector 310 moves.

The pulling device 320 is a device that functions to selectively pull the cuff connector 310, i.e. to tension the wrist cuff 100A, which is loosely worn around the wrist at normal times, to a certain degree or more for blood pressure measurement. The cuff tightener 300 may function to increase the peripheral length of the wrist-worn blood pressure monitor as well as to reduce the same, or may function only to reduce the peripheral length of the wrist-worn blood pressure monitor. For example, the pulling device may function to push the cuff connector as well as to pull the same, or may function only to pull the cuff connector.

Described in more detail, the pulling device 320 is provided at the wrist cuff 100A, for example, at the left strap 110B, and moves the cuff connector 310 in the forward direction, thereby reducing the peripheral length of the loop that is formed by the wrist-worn blood pressure monitor, specifically by the wrist cuff 100.

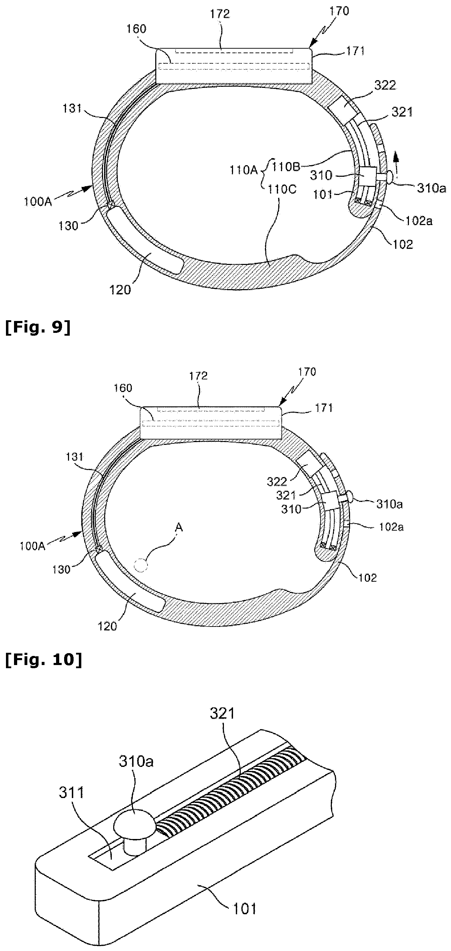

Therefore, as shown in FIG. 8, when the cuff tightener 300 is operated in the state in which the wrist-worn blood pressure monitor is worn a little loosely around the wrist of the examinee, the wrist cuff 100A is contracted to reduce the peripheral length thereof, as shown in FIG. 9, and consequently the wrist is compressed by the wrist cuff and a predetermined portion of the wrist, i.e. a portion corresponding to the radial artery A, is compressed by the air bag 120. Although it is illustrated in FIGS. 6, 8 and 9 that a portion at which the air bag 120 is provided has the same thickness as the remaining portion, the portion at which the air bag 120 is provided may swell to be thicker than the remaining portion.

When the cuff tightener 300 is operated in the reverse direction, the peripheral length of the wrist-worn blood pressure monitor is increased back to the state in FIG. 8 from the state in FIG. 9, and consequently the force of compressing the wrist is reduced.

As the result of the cuff connector 310 being alternately moved in the forward direction and in the reverse direction by the pulling device 320, the peripheral length of the wrist-worn blood pressure monitor is increased and reduced (the wrist-worn blood pressure monitor is expanded and contracted); however, the cuff connector 310 and the pulling device 320 may be detachably connected to each other.

For example, the cuff connector 310 may be connected to the pulling device 320 only when the cuff connector 310 is pulled in the forward direction by the pulling device 320, the cuff connector 310 may be disconnected from the pulling device 320 under a certain situation, for example, a situation in which the supply of power to the cuff tightener 300 is interrupted due to battery depletion or the like, the expansion and contraction of the wrist-worn blood pressure monitor may be performed manually in the state in which the cuff connector 310 and the pulling device 320 are disconnected from each other, and the wrist strap may be naturally loosened by the expansion of the wrist, around which the wrist-worn blood pressure monitor is worn.

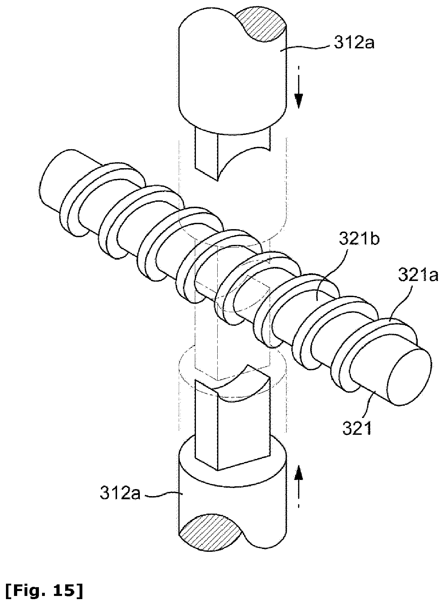

Referring to FIGS. 10 to 14, the pulling device 320 may include a pulling rod 321, which is connected to the cuff connector, and an actuator 322, such as a motor or the like, which drives the pulling rod 321 to move the cuff connector 310.

In this embodiment, the pulling rod 321 is configured as a bar that is rotated on its axis by the actuator 322, and when the pulling rod 321 is rotated, the cuff connector 310 is pulled along a movement passage formed in the wrist strap 110, i.e. along a slot-shaped connector movement passage.

Described in more detail, the pulling rod 321 in this embodiment is a screw bar that is rotatably inserted into the cuff connector 310, is rotated on its axis by the actuator 322, and has screw threads 321a formed in the outer circumferential surface thereof.

When the pulling rod 321 is rotated by the actuator 322, the cuff connector 310 is moved along the axis (in the longitudinal direction) of the pulling rod 321. That is, the rotational movement of the pulling rod 321 is converted into the linear movement of the cuff connector 310.

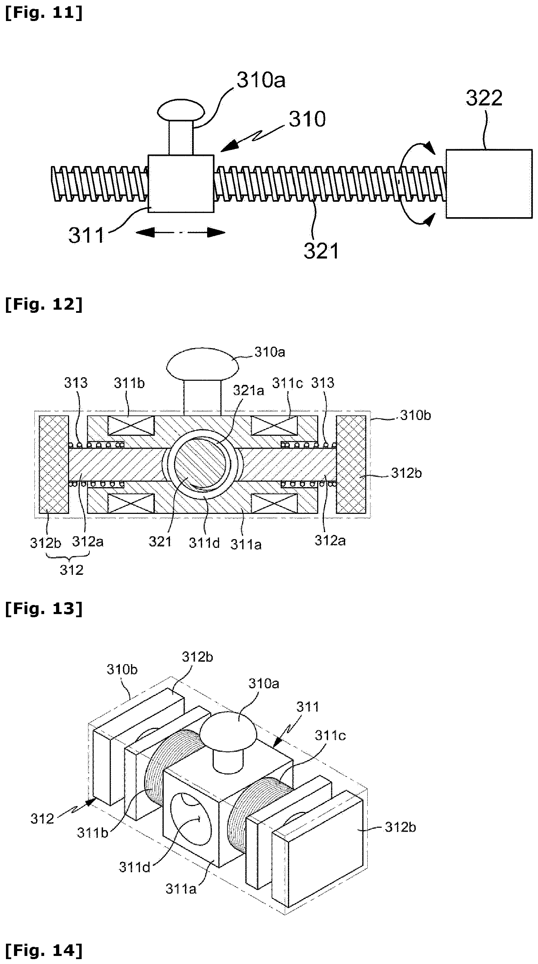

As shown in FIG. 12, the pulling rod 321 is configured to be rotated bidirectionally, i.e. the pulling device 320 may tighten and release (loosen) the wrist cuff, i.e. achieve expansion and contraction of the wrist-worn blood pressure monitor. However, the actuator may be embodied as a motor that is configured to be rotated unidirectionally, so that the pulling rod 321 is rotated only in the direction of pulling the cuff connector 310, and the wrist cuff may be manually released or may be naturally released by the expansion of the wrist, around which the wrist-worn blood pressure monitor is worn, without using additional power (electric power) after the pulling device 320, specifically the pulling rod 321, is disconnected from the cuff connector 310.

In this embodiment, the cuff connector 310 and the pulling rod 321 are detachably connected to each other. When the cuff connector 310 and the pulling rod 321 are in the connected state, i.e. in the engaged state, the cuff connector 310 may be moved unidirectionally or bidirectionally by the operation of the pulling device, and when the cuff connector 310 and the pulling rod 321 are in the disconnected state, i.e. in the disengaged state, kinetic energy is not transmitted from the pulling device 320 to the cuff connector 310, and consequently the cuff connector 310 may be moved freely without being influenced by the pulling device 320.

As described above, in order to detachably connect the cuff connector 310 and the pulling rod 321, the cuff connector 310 is selectively tooth-engaged with (meshes with) the pulling rod 321, and when the cuff connector 310 and the pulling rod 321 are in the tooth-engaged state, i.e. in the connected state, the cuff connector 310 may be moved by the pulling device 320.

In this embodiment, the cuff connector 310 includes a connector body 311, into which the pulling rod 321 is inserted, and a rod connection/disconnection member 312 for detachably connecting the pulling rod 321 and the connector body 311.

The connector body 311 is provided at one end portion 101 of the wrist cuff so as to move in the longitudinal direction of the wrist cuff 100A, and connects one end portion of the wrist cuff, i.e. the left strap 110B, to the opposite end portion 102 of the wrist cuff, i.e. to the right strap 110C.

The connector body 311 has a rod hole 311d formed therethrough in the anterior-posterior direction, into which the pulling rod 321 is inserted, and when the actuator 322 is driven in the state in which the rod connection/disconnection member 312 meshes with, i.e. is tooth-engaged with, the pulling rod 321, the connector body 311 is moved along the pulling rod 321.

The rod connection/disconnection member 312 functions as a clutch, which is movably provided at the connector body 311 for connection and disconnection between the cuff connector 310 and the pulling rod 321 and selectively meshes with the screw threads 321a in the pulling rod. In this embodiment, the connection between the pulling device 320, specifically, the pulling rod, and the cuff connector 310 is achieved by the supply of power to the cuff connector 310.

The connector body 311 in this embodiment is an electromagnet, and when the connector body 311 is magnetized, the cuff connector 310 and the pulling device 320 are connected to each other, and when the connector body 311 is demagnetized, the cuff connector 310 and the pulling device 320 are disconnected from each other.

Described in more detail with reference to FIGS. 12 to 14, the rod connection/disconnection member 312 is moved by the magnetic field that is induced in the connector body by the flow of the electric current. In other words, when electric current flows through the connector body 311, the connector body 311 is magnetized, and the rod connection/disconnection member 312 is moved by the magnetic force of the connector body 311 and meshes with the screw threads 321a in the pulling rod 321, more specifically with the valleys 321b between the screw threads.

The connector body 311 is a solenoid, which includes a frame 311a, in which the rod hole 311d is formed, and coils 311b and 311c, which are wound around the frame 311a. When electric current flows through the coils 311b and 311c, one end portion (e.g. the left portion) of the frame 311a, which is a conductor, is magnetized into the N-pole, and the opposite end portion (e.g. the right portion) of the frame 311a is magnetized into the S-pole. Since the principle of the electromagnetic solenoid is well known, further explanation thereof is omitted.

The coils 311b and 311c are wound around the left portion and the right portion of the frame 311a, respectively, and the left portion and the right portion of the frame 311a have coil-winding recesses formed therein. In this embodiment, the coils include a left coil 311b and a right coil 311c, which are wound around the left portion and the right portion of the frame 311a, respectively, and, as described above, the coils are wound around the frame 311a so that any one of the both end portions of the frame 311a is magnetized into the N-pole and the other end portion of the frame 311a is magnetized into the S-pole.

The rod connection/disconnection member 312 includes a connection/disconnection pin 312a, which selectively meshes with the screw threads in the pulling rod 321, and a base member 312b, which is provided at one end portion of the connection/disconnection pin 312a. The frame 311a has a rod connection/disconnection hole (i.e. a portion into which the connection/disconnection pin is inserted), into which the rod connection/disconnection member 312 is movably inserted.

The connection/disconnection pin 312a is configured as a non-magnetic body, which is movably inserted into the rod connection/disconnection hole, and the distal end of the connection/disconnection pin 312a is selectively tooth-engaged with the screw threads in the pulling rod 321. The base member 312b is configured as a conductor that can be attached to a magnet, i.e. as a magnetic body, and when the connector body 311 is magnetized, the base member 312b is moved by the magnetic force, and accordingly the connection/disconnection pin 312a is also moved together with the base member 312b and meshes with the pulling rod 321.

The rod connection/disconnection hole is formed through the frame 311a in the left-right direction. The rod connection/disconnection hole and the rod hole 311d intersect in a cross shape ('+' shape) and communicate with each other. The rod connection/disconnection hole may be divided into a first connection/disconnection hole, which is formed to the left of the rod hole 311d, and a second connection/disconnection hole, which is formed to the right of the rod hole 311d. In this embodiment, the rod connection/disconnection member 312 is mounted in the first connection/disconnection hole and in the second connection/disconnection hole.

When the supply of electric power to the electromagnet, more specifically to the coils 311b and 311c, is interrupted, the rod connection/disconnection member 312 is separated from the pulling rod 321, and consequently the connection, i.e. the engagement, between the connector body 311 and the pulling rod 321 is released. In this embodiment, the rod connection/disconnection member 312 is separated from the pulling rod 321 by the restoring force of the spring 313.

The spring 313 is provided in an inlet portion of the rod connection/disconnection hole so as to elastically support the base member 312b toward the outside of the frame 311a. Described in more detail, each spring 313 is provided in the corresponding inlet portion of the first connection/disconnection hole (i.e. the left portion of the frame) and the corresponding inlet portion of the second connection/disconnection hole (i.e. the right portion of the frame), so as to elastically support the base member 312b mounted to the left of the frame 311a and the base member 312b mounted to the right of the frame 311a in the left direction and the right direction of the frame, respectively.

When electric power is applied to the coils 311b and 311c, the force of attraction between the connector body 311 and the base member 312b moves the base member 312b against the spring 313, and the connection/disconnection pin 312a is moved into the frame 311a by the base member 312b, thereby connecting the pulling rod 321 and the connection/disconnection pin 312a.

On the other hand, when the supply of electric power to the coils 311b and 311c is interrupted, the base member 312b is pushed out by the spring 313, and consequently the connection/disconnection pin 312a is separated from the pulling rod 321.

Accordingly, the force of restraining the movement of the cuff connector 310 and the pulling force are eliminated, and the wrist cuff 100A may be naturally loosened by resilience (expansion) of the skin and subcutaneous tissue without using additional power.

Of course, as described above, the wrist cuff 100A may be untightened or loosened by the reverse operation of the pulling device 320 (rotation of the pulling rod in the reverse direction). However, in the case in which the pulling rod 321 and the connector body 311 are connected to each other at all times, when an abnormal situation, for example, battery depletion or the like, occurs while the wrist cuff 100A is compressing the wrist, the wrist cuff 100A may be continuously tightened and the wrist may thus be continuously compressed, impeding blood flow. In this embodiment, since the force of restraining the cuff connector 310 is eliminated when the supply of power is interrupted, it is possible to prevent the above problems.

In this embodiment, the connector body 311 and the rod connection/disconnection member 312 are accommodated in the connector case 310b, and the foregoing engagement protrusion 310a protrudes above the top surface of the frame 311a.

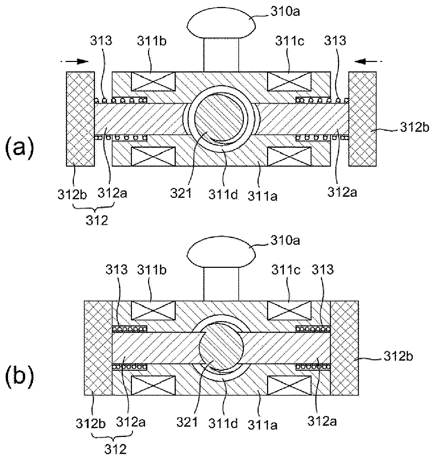

FIG. 15 is a view illustrating the connection and disconnection between the cuff connector 310 and the pulling rod 321, and when the connection/disconnection pin 312a of the rod connection/disconnection member and the pulling rod 321 are in the state of being separated from each other, as shown in FIG. 15a, the driving force of the actuator 322 is not transmitted to the cuff connector 310, and consequently the cuff connector 310 may be freely moved bidirectionally.

On the other hand, when the connection/disconnection pin 312a of the rod connection/disconnection member and the pulling rod 321 are in the state of being connected to each other, as shown in FIG. 15b, the driving force of the actuator 322 may be transmitted to the cuff connector 310; however, while the operation of the actuator 322 stops, the movement of the cuff connector 310 is restrained by the pulling rod 321. When the supply of power to the coils is interrupted, the connection/disconnection pin 312a and the pulling rod 321 are separated from each other by the spring 313, as shown in FIG. 15a, and consequently the wrist cuff may be naturally loosened.

Blood pressure measurement using the above-described wrist-worn blood pressure monitor may be performed in various manners such that the blood pressure measurement is conducted while the wrist of the examinee is being compressed by the wrist-worn blood pressure monitor or is conducted while the compressing force is being released as a result of the wrist-worn blood pressure monitor being loosened.

Additionally, the wrist-worn blood pressure monitor according to this embodiment (the second embodiment), like the wrist-worn blood pressure monitor depicted in FIG. 5, may further include a wireless communication unit for transmitting data of the measured blood pressure (the blood pressure of the user) to a predetermined terminal, for example, a smart phone, other kinds of terminals, or a management module.

Although exemplary embodiments of the present invention have been described, it will be apparent to those skilled in the art that various improvements, modifications, replacements, and additions can be made without departing from the scope and spirit of the invention.

Therefore, the above embodiments are to be construed as illustrative and not restrictive, and the present invention is not limited to the above description and may be modified and varied within the scope of the appended claims and their equivalents.

### [Industrial Applicability]

The present invention provides a portable wrist-worn blood pressure monitor that is worn on a wrist and is applicable to the field of medical devices or healthcare devices. According to the present invention, a user is able to conveniently measure his/her blood pressure whenever there is a need to measure the blood pressure, and the accuracy of the measured blood pressure is increased since the wrist-worn blood pressure monitor is securely tightened around the wrist.

## Claims

1. A wrist-worn blood pressure monitor configured to be worn around a wrist, comprising:
a wrist cuff (100) including a wrist strap (110) so as to be worn around a wrist; and
a sensor (130) provided at the wrist cuff (100) in order to measure blood pressure:
wherein the wrist strap (110) includes;
a strap frame (111) made of a hard material and formed to be bent in a predetermined shape so as to be mounted to a predetermined portion of the wrist and to be worn around the predetermined portion of the wrist; and
a connection strap (112) that is flexible and is secured to and supported by the strap frame.

2. The wrist-worn blood pressure monitor according to claim 1, wherein the connection strap (112) extends from one end of the strap frame (111) and is connected to an opposite end of the strap frame (111).

3. The wrist-worn blood pressure monitor according to claim 2, wherein one end of the connection strap (112) is secured to the strap frame (111), and an opposite end of the connection strap (112) is detachably connected to the opposite end of the strap frame (111).

4. The wrist-worn blood pressure monitor according to claim 3, wherein a tightener (200) is provided at the wrist cuff (100) in order to selectively tighten the wrist cuff (100) so that the wrist cuff compresses the wrist.

5. The wrist-worn blood pressure monitor according to claim 4, wherein the opposite end of the connection strap (112) and the opposite end of the strap frame (111) are connected to each other by the tightener (200).

6. The wrist-worn blood pressure monitor according to claim 1, wherein the strap frame (111) includes:
a base frame (111a) for supporting the connection strap; and
an auxiliary frame (111b) for height adjustment, the auxiliary frame being movably provided at the base frame (111a) in order to adjust a height of the wrist cuff to match a size of the wrist.

7. The wrist-worn blood pressure monitor according to claim 1, wherein the strap frame (111) has a curved shape so as to cover a portion corresponding to an ulnar artery.

8. The wrist-worn blood pressure monitor according to claim 1, wherein the wrist cuff (100) further includes an air bag (120) provided at the connection strap (112) in order to compress the wrist.

9. The wrist-worn blood pressure monitor according to claim 8, wherein the air bag (120) is connected with a valve (140) for injection of air thereinto.

10. The wrist-worn blood pressure monitor according to claim 9, wherein the valve (140) is provided at the air bag (120).

11. The wrist-worn blood pressure monitor according to claim 8, wherein the sensor (130) is provided at the air bag (120).

12. The wrist-worn blood pressure monitor according to claim 1, wherein the wrist cuff (100) further includes a display unit (170) for displaying the blood pressure, and wherein the display unit (170) is mounted to the strap frame (111).
